# EUROPEAN PATENT APPLICATION

(11) **EP 4 278 968 A1**
(43) Date of publication of application: **22.11.2023**
(21) Application number: 22174277.8
(22) Date of filing: 19.05.2022
(51) Int. Cl.: A61B 5/1455, A61B 5/00

(54) **LIGHT SHIELDING COVER AND SENSOR SYSTEM WITH LIGHT SHIELDING COVER**

(71) Applicant: Universität Zürich, 8006 Zürich (CH)
(72) Inventor: NÜSSLI, Anais, 9500 Will (CH); KLEISER, Stefan, 79639 Grenzach-Wyhlen (DE); REARTE, Andrea, 8006 Zürich (CH); KALYANOV, Alexander, 5507 Mellingen (CH); OSTOJIC, Daniel, 8046 Zürich (CH)
(74) Representative: Mertzlufft-Paufler, Cornelius

(57) **Abstract**

Light shielding cover (1) for an optical sensor (7) for measuring optical parameters in a scattering medium, wherein an active surface (8) of the optical sensor (7) is placed on a surface of the medium during measuring, the cover (1) comprising: a chamber (2) for inserting an optical sensor (7), wherein the chamber (2) comprises an opening (3) through which the active surface (8) of an inserted optical sensor (7) is accessible, and a sealing lip (4) surrounding the chamber (2), wherein the sealing lip (4) protrudes from a rim (5) of the chamber (2) in a direction normal to and away from the active surface (8) of an inserted sensor (7), and wherein the circumference of the sealing lip (4) at a contact area (6) is larger than the circumference of the chamber (2).

## Description

The invention concerns light shielding cover for an optical sensor for measuring optical parameters in a scattering medium, wherein an active surface of the optical sensor is placed on a surface of the medium during measuring.

The invention relates to the field of spectrophotometry and oximetry, which aims at measuring optical properties and deriving parameters such as oxygen saturation in living tissue. Spectrophotometry allows determination of optical properties such as absorption and scattering coefficients by illuminating a specimen and determining the attenuation in light intensity over distance. Oximeters are apparatuses that make use of spectrophotometry for determining the (arterial, venous or mixed) oxygenation of blood in tissue.

In particular, the invention relates especially to the measurement of cerebral hemoglobin saturation in heads, especially of neonates or small children. The heads of neonates or small children are very small and therefore have a small radius and strong curvature. It is thus necessary to fix the active surface of the sensor to the head using a bandage or other means. But neonates also have very thin and sensitive skin. When fixing the sensor to the head, excessive forces and pressure on the skin should be avoided.

It is an object of the present invention to improve usage properties of an optical sensor.

In accordance with a first embodiment of the present invention, the object is solved by a light shielding cover according to claim 1.

According to the invention the light shielding cover comprises a chamber for inserting an optical sensor, wherein the chamber comprises an opening through which the active surface of an inserted optical sensor is accessible, and a sealing lip surrounding the chamber, wherein the sealing lip protrudes from the chamber in a direction normal to and away from the active surface of an inserted sensor (towards the patient) and wherein the circumference of the sealing lip at the distal portion forming a contact area is larger than the circumference of the chamber.

The sealing lip may be formed adjacent to a rim of the chamber and thus protrude from that rim.

Such an optical sensor comprises in general one or more light emitting sources and one or more light receivers, placed distanced away from the light emitting source. To perform a measurement, a sensor is inserted into the chamber of the light shielding cover according to the invention. The sensor with the cover is then placed on the surface of the medium to be measured. The medium could be for example cerebral tissue of a neonate, in which case the sensor with cover is placed on the head of the neonate.

When placed on the surface, for example the head of a neonate, the active surface of the sensor is brought into contact with the surface of the medium through the opening in the chamber. The sealing lip, which protrudes away from the active surface, that means in the direction towards the surface of the medium, tightly seals the chamber and shields the sensor from ambient light. Thus, the sensitivity and accuracy of the measurement can be increased since the signal to noise ratio is increased.

Even on surfaces with small radii, such as for example a head of a neonate, where a sensor would be strongly curved and may not fit snugly on the head, the sealing lip will tightly shield the sensor from ambient light.

Since the circumference of the sealing lip is larger than the circumference of the chamber, and an inserted sensor, it also reduces the amount of light that reaches the sensor(s) by entering the skin next to the sensor and percolating the tissue or skin to the sensor.

The light shielding cover has the overall effect, that less force is needed to fit it tightly to the surface. The usage properties, when using the invention on neonates is thus improved.

In accordance with another embodiment of the present invention, the object is solved by a light shielding cover according to claim 2.

In the embodiment the cover comprises a chamber for inserting an optical sensor, wherein the chamber comprises an opening through which the active surface of an inserted optical sensor is accessible, wherein the chamber has one or more relief portions to accommodate protrusions or stiffer portions of an optical sensor, wherein a relief portion comprises a recess in the inside and/or the outside of the chamber wall associated with the location(s) of the protrusion(s) or stiffer portion(s) of an inserted sensor.

As described earlier, a sensor comprises at least a light source and a receiver. These parts are usually stiffer than for example a surrounding portion of the casing and they directly or indirectly (via for example a filter or window) contact a surface of a medium. That means, when applied to for example the head of a neonate, these stiffer portions can cause pressure points on the skin of the head which may lead to damage of the skin.

The relief portions have the effect, that pressure applied to the cover, for example by a fixating bandage, is not directed to these stiffer portions of the sensor. This greatly reduces or eliminates indentations of the stiffer portions into the skin. Neonates normally are monitored during longer periods so that the elimination of such indentations is a vast improvement of the usage properties of a sensor.

In an aspect the chamber has a counterpart form of an optical sensor to be housed. This has the advantage that the cover sits tightly on the sensor and pressure applied on the cover is well distributed and passed on to the sensor. Therefore, a tight seat on a surface of the sensor and the cover is provided. Moreover, if relief portions are present, the pressure applied on the cover is distributed to non-protruding portions and or less stiff portion(s) of the sensor.

Alternatively or additionally, the recess of one or more relief portions comprise an through opening in the chamber wall. Preferably any protrusion or stiffer part of the sensor does not project from the opening to the outside of the chamber. This has the effect that any fixation means, like a bandage, does not apply pressure on the stiffer part(s). The risk of excessive pressure or the forming of indentation on the surface of the skin is reduced.

In one aspect, the sealing lip comprises a bending area. The bending area allows the sealing lip to be compresses or at least deflect or move more easily relative to the chamber. Preferably the deflection is facilitated in a direction normal to a surface where the sealing lip is placed on. However, other deflecting directions could be possible. The bending area thus enables the sealing lip for example to adapt to different shapes, sizes and curvatures of heads and provides tight shielding from ambient light. It is possible that the sealing lip comprises more than one bending area.

Preferably the sealing lip extends circumferentially around at least a portion of chamber, preferably around at least 25% of the circumference of the chamber.

Preferably the bending area extends circumferentially around at least a portion of chamber, preferably around at least 25% of the circumference of the chamber and or the bending area extends circumferentially around only a portion of the circumference of the chamber, preferably around less than 75% of the circumference of the chamber.

In an example, the sealing lip comprises a circumferential step. The step can constitute or be part of the bending area or it can be distal thereof.

In another example, the sealing lip has an S-shaped cross section, wherein at least one bend of the S-shape constitutes the bending area. Preferably the other bend of the S-shape is part of the sealing lip.

As another example, the sealing lip is formed in the form of a gaiter or bellow, wherein the S-shaped cross section could provide a gaiter function.

The above-mentioned examples merely describe some possible embodiments of the sealing lip without the invention being limited to the examples. Also, these examples are not mutually exclusive but could be combined in any way.

Typically, a sensor has a rectangular shape with a long side and a short side. Axial in the context of the invention means parallel to the long side of the sensor.

In an aspect, the contact area of the cover has a concave cross section, at least in an axial direction of the cover form. The concave form enables the cover to fit on small objects or surfaces with strong curvature and still provides shielding from light.

In an aspect, the toe of the sealing lip is inclined with respect to the plane of the active surface of a sensor, especially wherein the inclination angle is between 20 and 60 degrees, preferably tapering in radial direction of the cover towards the sensor. The inclined surface provides a larger contact area on a surface and thus provides better shielding. It also provides softer skin contact. The inclination angle may be adapted to different curvatures of the body part. It may be advantageous to provide different covers with varying inclination angles for different curvatures. Therefore, a cover may be selected according to an actual use case.

In an aspect, the sealing lip comprises a chamfer in axial direction or a circumferential direction, especially wherein a height of the sealing lip is essentially zero at the base of the chamfer. A circumferential direction is a direction parallel to a side of the chamber. That means the sealing lip varies in its height along such a direction. The hight is measured in the direction normal to the rim of the chamber.

It is especially beneficial, if the chamfer is oriented in a way that the sealing lip has its maximum height in a region where a receiver of an inserted sensor is seated. Accordingly in a region where a light source of an inserted sensor is seated, the height of the sealing lip could be zero since no light shielding is required there for proper measurements.

Due to the chamfer in the sealing lip, the cover is less stiff in an axial direction and facilitates the sealing of small heads or strong curvatures. Moreover, if the sealing lip does not run completely around the chamber, the pressure required to form or fit the chamber to the patient is further reduced.

The chamfer also saves material and the cover may be cheaper to manufacture.

In an aspect, the sealing lip is asymmetrically arranged around the chamber. That means the sealing lip does not fully encircle the circumference of the chamber. In other words, there is at least a region in the circumference of the chamber, where the height of the sealing lip is zero or nearly zero.

This may provide better sealing on surfaces with strong curvature. The asymmetrical sealing lip can be formed by a chamfer on one or both long sides of the chamber in an axial direction. This is especially beneficial when the sensor is also asymmetrical, that means when the light sources are placed on one side of the active surface only.

In one aspect, the chamber comprises a circumferential recessed rim where a counterpart rim of an optical sensor bears on. This has the effect that the cover has a surrounding contact area to the sensor which may improve or enable a uniform pressure distribution.

In an aspect the cover comprises means for fixing a sensor in the chamber. By this the sensor is held in the cover even when not fixed to a surface. Such fixing means may improve the handling and usability of a sensor.

In an aspect, the cover comprises as fixing means an undercut where a rim of the sensor could be inserted or that embraces a part of the sensor.

In another aspect, the cover comprises as fixing means a strap that spans between two opposing sides or rims of the chamber. Preferably the sensor comprises a trench or recess where this strap is seated when the sensor is inserted in the chamber, so that the strap does not project from the plane of the active surface.

In an aspect, the cover comprises a channel to accommodate a cable from an inserted sensor, wherein the cover, preferably in the region of the channel, comprises at least one region with increased flexibility. The flexible region in the channel reduces force or torque that the cable exerts on the cover. The cover stays tightly seated on a surface even when the cable is moved.

The flexible area may comprise thinner or softer material or may be formed by a slit alongside the cable. Preferable two slits are formed alongside the cable.

In one aspect, the cover comprises a channel to accommodate a cable from an inserted sensor, the channel being open on the side of the opening of the chamber, the channel reaching through the sealing lip and into the chamber, the channel comprising channel walls perpendicular to the chamber opening and the sealing lip and a channel ceiling opposite of the opening and wherein the channel ceiling comprises slits along the inside of the chamber walls. The channel walls extend to the sealing lip, so that they provide sealing of the channel on a surface.

In an aspect, the cover is made of an elastic and/or flexible material, preferably silicone or a TPE like TPU, and/or wherein the material of the cover has a high absorption in the UV, visible, infrared and near-infrared spectrum. By this the cover is cost-efficient and easy to manufacture, for example using injection moulding.

In an aspect, the contact area of the sealing lip is sticky. A sticky surface may improve the light shielding and provides better seat on a surface.

Additionally or alternatively the contact area may comprise an adhesive that adheres the sealing lip to a surface. The adhesive may be applied partially or completely. The adhesive may be sufficient to fix the sensor and the cover, so that additional fixating means, like bandages, are not necessary.

The object is also solved by a sensor system comprising an optical sensor for measuring optical parameters in a scattering medium and a light shielding cover according to the invention, wherein the sensor is removably insertable into the chamber of the cover. A removable cover has the advantage that it could be applied only where it is needed and thus keeps the sensor simple and cheap. A removable cover is also cheaper and may be easier to clean or even could be sterilized. A removable cover could also be made disposable and for single use only.

The object is further solved by a sensor system comprising an optical sensor for measuring optical parameters in a scattering medium and a light shielding cover according to the invention, wherein the light shielding cover is integrally formed with the sensor. For some applications it may be beneficial that the cover is integrally formed with the sensor. The integral cover is always in the correct position, which may facilitate the application of the sensor system.

Preferred embodiments of the invention are described with reference to the attached figures. The shown embodiments are exemplary only and not limiting in any way.
- Fig. 1:: a perspective view from above of a light shielding cover according to an embodiment,
- Fig. 2:: a perspective view from below of a light shielding cover,
- Fig. 3: a side view of a long side of the cover of Fig. 1 with a cable channel,
- Fig. 4:: a side view of the opposing long side of Fig. 3,
- Fig. 5: a side view of a short side of the cover of Fig. 1,
- Fig. 6:: a side view of the opposing short side of Fig. 6,
- Fig. 7:: a top view of the cover of Fig. 1,
- Fig. 8:: a sectional view of an axial section of a sensor system with a cover of Fig. 1 and a sensor inserted in the cover,
- Fig. 9:: a perspective view of Fig. 8,
- Fig. 10:: a sectional view of a sensor system according to another embodiment.

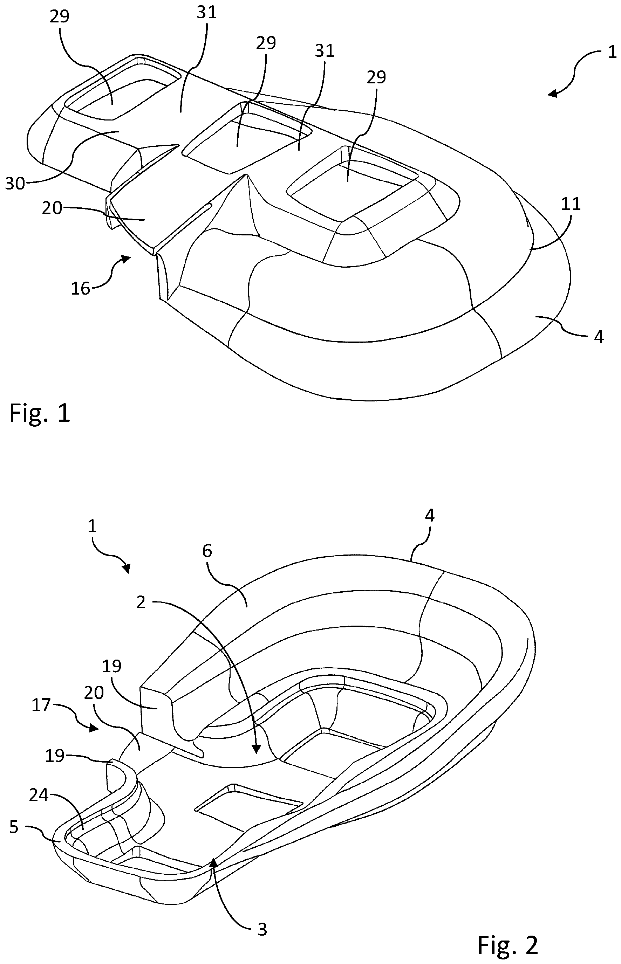

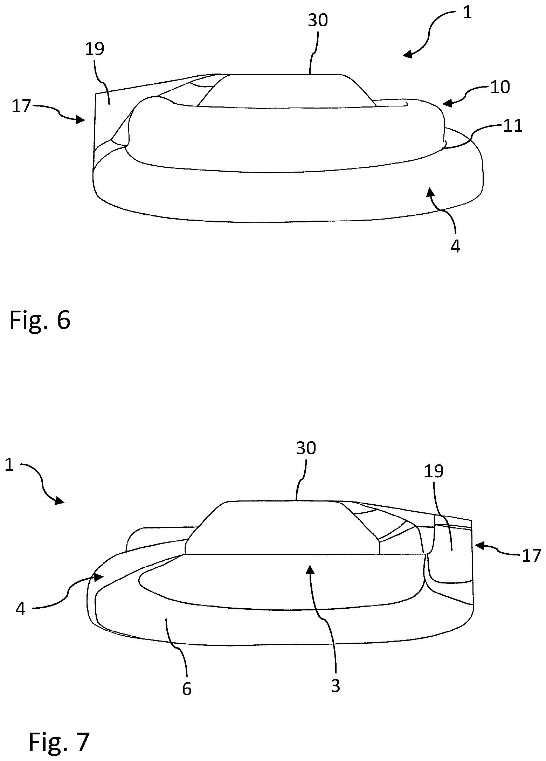

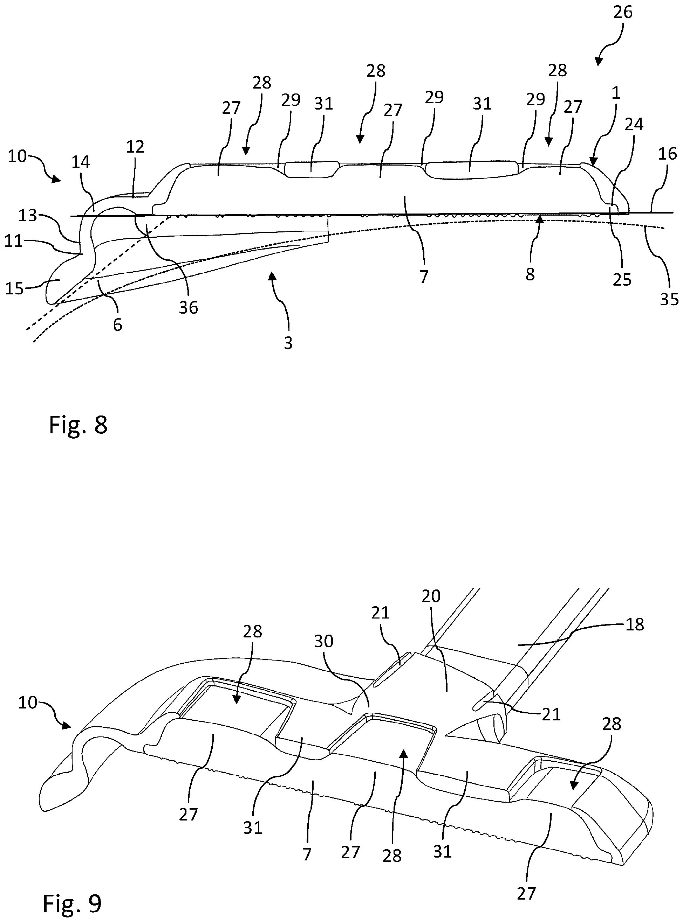

Figures 1 to 7 show a light shielding cover 1 according to a first embodiment of the invention. Figures 8 and 9 show the cover 1 with a sensor 7 inserted in the cover 1. The first embodiment is described with respect to these figures 1 to 9 with no further reference to a specific figure.

The cover 1 comprises a chamber 2 for inserting an optical sensor 7, wherein the chamber 2 comprises an opening 3 through which the active surface 8 of an inserted optical sensor 7 is accessible. In the active surface 8 of the sensor 7 light sending and receiving means are placed. The opening 3 may also be used to insert the sensor 7 into the chamber 3.

The cover 1 further comprises a sealing lip 4 surrounding the chamber 2, wherein the sealing lip 4 protrudes from a rim 5 of the chamber 2 in a direction 9 normal to and away from the active surface 8 of an inserted sensor 7. The circumference of the sealing lip 4 at a contact area 6 is larger than the circumference of the chamber 2.

In this exemplary embodiment the chamber 2 is dimensioned to accommodate a rectangular sensor 7, with a long side (length) and a short side (width). The sensor and thus the chamber 2 are for example 4 cm long and 1 cm wide.

However different sensor can have differing sizes. The cover is preferably adapted to a specific size of a sensor 7 to be housed in the chamber 2. The invention is thus by no means limited to the mentioned dimensions.

The chamber 2 comprises a circumferential recessed rim 24 where a counterpart rim 25 of an optical sensor 7 bears on.

The sealing lip 4 comprises a bending area 10, where the sealing lip 4 is movable or deflectable relative to the chamber 2. For example, a deflection is enabled in the direction 9 normal to the active surface 8. The sealing lip 4 comprises a bending area 10 to enable such deflection or movement.

The bending area 10 is constituted of a first part 12, that extends from the rim 5 parallel to the plane 13 of the active surface 8 and a second part 14, that extends normal to the plane 16, wherein the first part 12 and second part 13 are connected by a circular, 90° bend 14.

Adjacent to the second part 14 of the bending area 10, the sealing lip 4 comprises a contact part or toe 15. The contact part 15 has a thicker wall than the bending area 10. The contact part 15 also comprises the contact area 6 on the inside of the sealing lip 4. The contact area 6 in the example is inclined to the plane 16. The inclination angle 36 here is around 40°. The inclination angle may be in the range of 20° to 60°.

Between the second part 14 and the contact part 15 a circumferential step 11 is formed resulting in an S-shaped cross section of the sealing lip. This also enables the sealing lip 4 to move like a gaiter.

Overall, the cover 1 has a concave form 35 that fits for example small heads or strong curved surfaces, see fig. 8.

The light shielding cover 1 comprises a channel 17 to accommodate a cable 18 from an inserted sensor 7. The channel 17 is open on the side of the opening 3 of the chamber 2. The channel reaches through the sealing lip 4 and into the chamber 2. This facilitates insertion of the sensor 7 with an attached cable 18 into the chamber 2.

The channel 18 comprises channel walls 19 that extend perpendicular to the chamber opening 2 and the sealing lip 4. The channel also comprises a channel ceiling 20 opposite of the opening and wherein the channel ceiling 20 comprises two slits 21 along the inside of the chamber walls 19. These slits 21 provide a region with increased flexibility. In use, when the cable 18 is moved, the slits enable the channel ceiling 20 to easily deflect.

Instead of the slits, that reach through the channel ceiling, only shallow trenches could be applied, so that thin membranes are formed in the trenches that allow deflection of the channel ceiling 20 as well while still closing off the channel.

In the shown example, the sealing lip 4 comprises a chamfer 22 in the direction of a long side of the chamber 2. The height of the sealing lip 4 is essentially zero at the base of the chamfer 22. Here the chamfer 22 results in the sealing lip being arranged asymmetrically around the chamber 2.

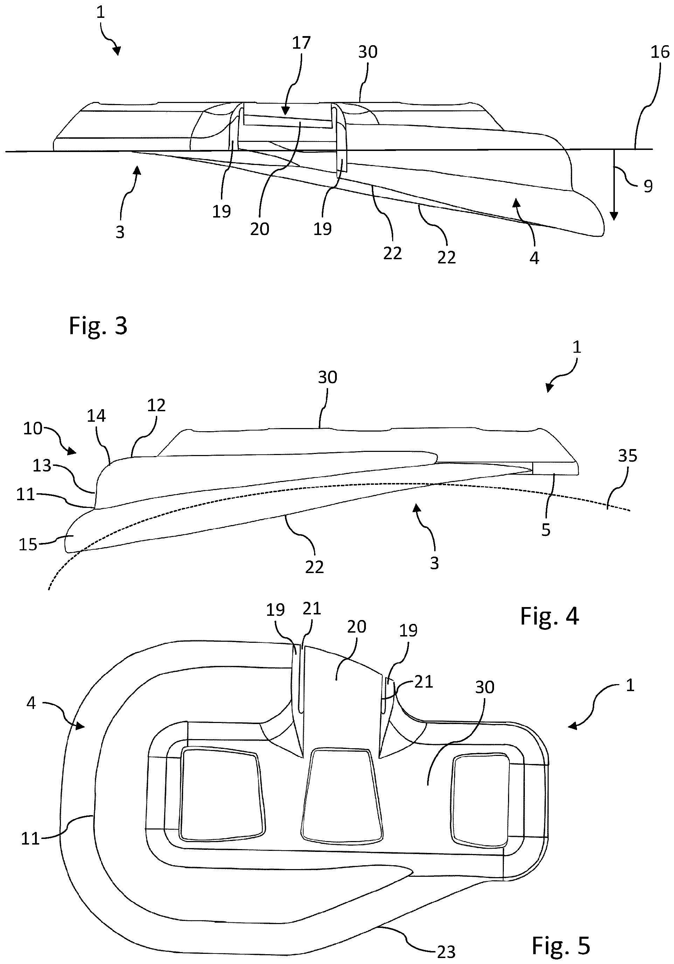

As could be seen in Fig. 5, the sealing lip 4 also comprises a chamfer 23 in a direction radial to the circumference of the chamber 2.

Fig. 8 and 9 show sensor systems 26 with a sensor 7 and a light shielding cover 1, wherein the sensor 7 is removably inserted in the cover 1. The sensor 7 in the example has three stiffer portions that comprise protrusions 27 where the sensor 7 is thicker than between these protrusions. In the region of these protrusions 27 or stiffer portions the light source and receivers are located.

The chamber 2 of the cover 1 here has a counterpart form of the sensor 7, so that the cover 1 tightly fits the sensor 7. The chamber 2 in the example comprises three relief portions 28 to accommodate these protrusions 27 of the optical sensor 7. In the shown example, these relief portions 28 are formed by openings 29 in the cover wall 30. However, the protrusions 27 of the sensor 7 do not project through the openings 29 to the outside of the chamber 2 but are recessed in the openings 29.

The cover wall 30 between these openings 28 form bars 31 that rest on the sensor surface. When fixing the sensor system 26 to a surface, for example to a head with a bandage, the bandage only contacts the cover wall 30 but not the protrusions 27 of the sensor 7. The force from the bandage is thus only applied to the bars 31. The bars 31 exert the force on the softer parts of the sensor but not to the stiffer parts or protrusions 27. The advantage here is that stiffer or rigid parts of the sensor, such as a light source or receiver is not pressed on the soft skin of for example a neonate.

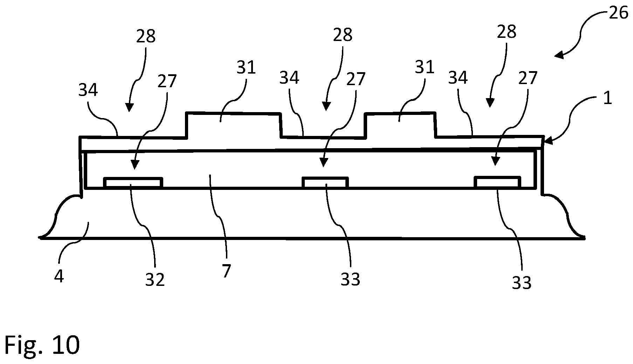

Fig. 10 shows another embodiment of a sensor system 26. Here the sensor 7 also has a light source 32 and two receivers 33 spaced apart. The light source 32 and receiver 33 form stiffer portions 27 of the sensor.

Different from the embodiment shown in figures 1 to 9, the sensor 7 does not have protrusions in the stiffer portions 27 but has a flat surface. Hence the inner wall of the chamber is also flat to sit tightly on the sensor.

Nonetheless, the cover comprises three relief portions 28 that are formed by recesses 34 in the outside of the cover wall 30. Between the recesses 34 bars 31 are formed. In this embodiment force exerted from a bandage only is applied to the bars 31 that pass the force to the sensor outside the stiffer portions 27.

In Fig. 10 the recesses 34 could be on the inside of the channel wall or a combination of varying measures could be applied. Therefore, the invention is not limited to the shown examples.

In this embodiment the sealing lip 4 does not comprise a chamfer. Hence the sealing lip 4 has a uniform height and completely surrounds the chamber 2.

Apart from the two shown embodiments, there are numerous ways to form the relief portions. However important is that at the relief portions prevent force getting applied to stiffer portions of the sensor 7.

### List of reference numbers

- 1: light shielding cover
- 2: chamber
- 3: opening
- 4: sealing lip
- 5: rim of chamber
- 6: contact area of sealing lip
- 7: sensor
- 8: active surface of sensor
- 9: direction normal to active surface of sensor
- 10: bending area
- 11: step
- 12: first part
- 13: second part
- 14: bend
- 15: contact part
- 16: plane of active surface
- 17: channel
- 18: cable
- 19: channel wall
- 20: channel ceiling
- 21: slit
- 22: chamfer in circumferential direction
- 23: chamfer normal to circumferential direction
- 24: rim in chamber
- 25: rim at sensor
- 26: sensor system
- 27: stiffer portion/protrusion
- 28: relief portion
- 29: opening
- 30: cover wall
- 31: bar
- 32: light source
- 33: receiver
- 34: recess
- 35: concave form
- 36: inclination angle

## Claims

1. Light shielding cover (1) for an optical sensor (7) for measuring optical parameters in a scattering medium, wherein an active surface (8) of the optical sensor (7) is placed on a surface of the medium during measuring, the cover (1) comprising:
a chamber (2) for inserting an optical sensor (7),
wherein the chamber (2) comprises an opening (3) through which the active surface (8) of an inserted optical sensor (7) is accessible,
and a sealing lip (4) surrounding the chamber (2), wherein the sealing lip (4) protrudes from the chamber (2) in a direction normal to and away from the active surface (8) of an inserted sensor (7), and wherein the circumference of the sealing lip (4) at a contact area (6) is larger than the circumference of the chamber (2).

2. Light shielding cover (1) according to the preamble of claim 1, preferably according to claim 1, the cover (1) comprising:
a chamber (2) for inserting an optical sensor (7),
wherein the chamber (2) comprises an opening (3) through which the active surface (8) of an inserted optical sensor (7) is accessible,
wherein the chamber (2) comprises one or more relief portions (28) to accommodate protrusions (27) or stiffer portions (27) of an optical sensor (7), wherein a relief portion (28) comprises a recess (34) in the inside and/or the outside of the chamber wall (30) at the location of the protrusion (27) or stiffer portion (27) of a sensor.

3. Light shielding cover according to claim 2, wherein the chamber (2) has a counterpart form of an optical sensor (7) to be housed and/or wherein the recess (34) of one or more relief portions (28) comprises an opening (29) in the chamber wall (30).

4. Light shielding cover according to one of the preceding claims, wherein the sealing lip (4) comprises a bending area (10) and/or wherein the sealing lip (4) comprises a circumferential step (11) and/or wherein the sealing lip (4) has an S-shaped cross section.

5. Light shielding cover according to one of the preceding claims, wherein the cover (1) has a concave cross section, at least in an axial direction of the cover (1).

6. Light shielding cover according to one of the preceding claims, wherein the contact area (6) of the sealing lip (4) is inclined with respect to the plane (16) of the active surface (8), especially wherein the inclination angle (36) is between 20 and 60 degrees, preferably tapering in an axial direction of the cover (1).

7. Light shielding cover according to one of the preceding claims, wherein the sealing lip (4) comprises a chamfer (22) in a circumferential direction, especially wherein a height of the sealing lip (4) is essentially zero at the base of the chamfer (22).

8. Light shielding cover according to one of the preceding claims, wherein the sealing lip (4) is asymmetrically arranged around the chamber (2).

9. Light shielding cover according to one of the preceding claims, wherein the chamber (2) comprises a circumferential recessed rim (24) where a counterpart rim (25) of an optical sensor (7) bear on.

10. Light shielding cover according to one of the preceding claims, wherein the cover (1) comprises a channel (17) to accommodate a cable (18) from an inserted sensor (7), wherein the cover (1), preferably in the region of the channel (17), comprises at least one region with increased flexibility, such as a slit (21) alongside the cable.

11. Light shielding cover according to claim 10, wherein the channel (17) is open on the side of the opening (3) of the chamber (3), the channel (17) reaching through the sealing lip (4) and into the chamber (2), the channel (17) comprising channel walls (19) perpendicular to the chamber (2) opening (3) and the sealing lip (4) and a channel ceiling (20) opposite of the opening (3) and wherein the channel ceiling (20) comprises slits (21) along the inside of the chamber walls (19).

12. Light shielding cover according to one of the preceding claims, wherein the cover (1) is made of an elastic material, preferably silicone or a TPE like TPU, and/or wherein the material of the cover has a high absorption in the UV, visible, infrared and near-infrared spectrum.

13. Light shielding cover according to one of the preceding claims, wherein the contact area (6) of the sealing lip (4) is sticky and/or comprises an adhesive.

14. Sensor system (26) comprising an optical sensor (7) for measuring optical parameters in a scattering medium and a light shielding cover (1) according to one of the previous claims, wherein the sensor (7) is removably insertable into the chamber (2) of the cover (1).

15. Sensor system (26) comprising an optical sensor (7) for measuring optical parameters in a scattering medium and a light shielding cover (1) according to one of the previous claims, wherein the light shielding cover (1) is integrally formed with the sensor (7).
